# EUROPÄISCHE PATENTANMELDUNG

(11) **EP 0 938 927 A1**
(43) Veröffentlichungstag der Anmeldung: **01.09.1999**
(21) Anmeldenummer: 99101427.5
(22) Anmeldetag: 27.01.1999
(51) Int. Cl.: B01J 31/04, C07C 41/03, C07C 67/26, C07C 69/24, C07C 69/52

(54) **Alkoxylierungskatalysator**

(30) Priorität: 26.02.1998 DE 19807991
(71) Anmelder: Clariant GmbH, 65929 Frankfurt am Main (DE)
(72) Erfinder: Wimmer, Ignaz, 84543 Winhöring (DE); Freundl, Hildegard, 84508 Burgkirchen (DE)

(57) **Zusammenfassung**

Alkoxylierungskatalysator, hergestellt durch
a) Umsetzung von einem Mol einer Dicarbonsäure, mit 1,5 bis 8 mol eines oxalkylierten Alkohols, unter Bildung des entsprechenden Dicarbonsäurehalbesters,
b) Bildung eines Erdalkali-Salzes des Dicarbonsäurehalbesters durch Zugabe von Wasser und 0,45 bis 0,55 mol einer basischen Erdalkaliverbindung pro Mol ursprünglich eingesetzter Dicarbonsäure,
c) Teilneutralisation des Erdalkali-Salzes durch Zugabe von 0,25 bis 0,7 mol H₂SO₄ pro Mol ursprünglich eingesetzter basischer Erdalkaliverbindung und
d) Entfernung des vorhandenen Wassers bei einer Temperatur unter 100°C.

Der so hergestellte Katalysator ermöglicht die Herstellung farbloser Oxalkylate mit enger Homologenverteilung.

## Beschreibung

Aus EP-A-337 239 ist bereits die Verwendung von Erdalkalisalzen von Monoestern aus Dicarbonsäuren und oxalkylierten Alkoholen als Katalysatoren zur Herstellung von "narrow-range''-Alkoxylaten bekannt. EP-A-657 410 beschreibt die Verwendung von Erdalkalisalzen von Monoestern aus Alkyl- oder Alkenylbernsteinsäure und oxalkylierten Alkoholen für den gleichen Zweck. In beiden Fällen ist die nicht veresterte Carboxylgruppe vollständig in die Form des Erdalkalisalzes überführt. Es wurde nun gefunden, daß man die Wirkung dieses Typs von Katalysator noch verbessern kann, wenn man die Carboxylat-Gruppe nur teilweise in die Salzform überführt.

Derartige teilneutralisierte Ca-Salze von Bernsteinsäurehalbestern und deren Verwendung als Alkoxylierungskatalysator sind bereits in US-5 600 020 beschrieben. Diese Bernsteinsäurehalbester sind jedoch durch einen C₈-C₃₀-Alkyl-rest substituiert. Oxalkylierungskatalysatoren der oben beschriebenen Art auf der Basis von Alkylbernsteinsäurehalbestern fallen jedoch als farbige Produkte an und geben gefärbte Oxalkylierungsprodukte. Die im folgenden beschriebenen Oxalkylierungskatalysatoren geben dagegen weitgehend farblose Oxalkylierungsprodukte mit enger Homologenverteilung.

Gegenstand der Erfindung ist ein Oxalkylierungskatalysator, der hergestellt wird durch
a) Umsetzung von einem Mol einer Dicarbonsäure der Formel

   HOOC-(CH₂)ₐ-COOH,

   wobei a eine Zahl von 1 bis 4 bedeutet, mit 1,5 bis 8 mol eines Alkohols der Formel

   R-(OA)ₓ-OH

   worin R C₄-C₂₂-Alkyl, C₄-C₂₂-Alkenyl oder eine Gruppe der Formel F-(CF₂)ₙ-(CH₂)ₘ-, n eine ganze Zahl von 6 bis 16, m eine ganze Zahl von 1 bis 4, x eine Zahl von 0 bis 6 und A -C₂H₄- oder -C₃H₆- bedeutet, unter Bildung des entsprechenden Dicarbonsäurehalbesters,
b) Bildung eines Erdalkali-Salzes des Dicarbonsäurehalbesters durch Zugabe von Wasser und 0,45 bis 0,55 mol einer basischen Erdalkaliverbindung pro Mol ursprünglich eingesetzter Dicarbonsäure,
c) Teilneutralisation des Erdalkali-Salzes durch Zugabe von 0,25 bis 0,7 mol H₂SO₄ pro Mol ursprünglich eingesetzter basischer Erdalkaliverbindung und
d) Entfernung des vorhandenen Wassers bei einer Temperatur unter 100 °C.

Die Herstellung des Dicarbonsäurehalbesters erfolgt nach an sich bekannten Methoden, wie z. B. in US 5 600 020 beschrieben. Die Reaktion erfolgt im allgemeinen bei 70 bis 140 °, vorzugsweise 80 bis 110 °C. Das Molverhältnis Alkohol zu Dicarbonsäure beträgt ungefähr 1,5 : 1 bis 8 : 1, bevorzugt 3 : 1 bis 6 : 1. Anstelle der Dicarbonsäure kann man naturgemäß auch von dem entsprechenden Säureanhydrid ausgehen. Bevorzugt ist Bernsteinsäureanhydrid. Bei den Alkoholen der Formel R-(OA)ₓ-OH ist A vorzugsweise -C₂H₄-.

Im zweiten Reaktionsschritt wird der saure Halbester durch Zugabe einer basischen Erdalkaliverbindung, vorzugsweise die Oxide-, Carbonate- oder Hydroxide von Barium, Strontium, insbesondere Calcium, in das Salz dieser Erdalkalimetalle überführt. Die Menge an basischer Erdalkaliverbindung beträgt ungefähr 0,45 bis 0,55, vorzugsweise 0,5 mol pro Mol des sauren Halbesters bzw. pro Mol Dicarbonsäure bzw. Dicarbonsäureanhydrid. Um die Salzbildung zu erleichtern, gibt man Wasser hinzu. Die Menge an Wasser ist unkritisch, aus praktischen Gründen arbeitet man im allgemeinen mit einer Wassermenge, die gleich der Menge an basischer Erdalkaliverbindung ist oder bis zum dreifachen davon beträgt. Höhere Mengen an Wasser bringen keinen Vorteil und erhöhen lediglich die Menge an Wasser, das im letzten Schritt wieder entfernt werden muß. Zur Vervollständigung der Salzbildung rührt man die Mischung im allgemeinen mehrere Stunden bei 60 bis 100, vorzugsweise 80 bis 95 °C.

Die Teilneutralisation erfolgt durch Zugabe von H₂SO₄ in der angegebenen Menge bei 20 bis 80, vorzugsweise 40 bis 70 °C. Bevorzugt arbeitet man mit 0,3 bis 0,6 mol H₂SO₄ pro Mol ursprünglich eingesetzter basischer Erdalkaliverbindung.

Im letzten Schritt wird das vorhandene Wasser im Vakuum abdestilliert, wobei die Temperatur 100 °C nicht überschreiten soll. Vorzugsweise entfernt man das Wasser im Vakuum 1 bis 5 Stunden lang bei 50 bis 100 °C, vorzugsweise bei 70 bis 90 °C. Man erhält auf diese Weise den Oxalkylierungskatalysator in Form einer weißen, mehr oder wenigen viskosen Slurry, die die nicht umgesetzte überschüssige Menge an Alkohol der Formel R-(OA)ₓ-OH enthält. Diese Slurry kann direkt bei Oxalkylierungsreaktionen als Katalysator eingesetzt werden.

Die Alkoxylierung, das heißt die Reaktion von Verbindungen mit aktiven H-Atomen mit Alkylenoxiden mit Hilfe der erfindungsgemäßen Produkte als Katalysator wird in üblicher Weise durchgeführt, das heißt bei einer Temperatur von 60 bis 200 °C, vorzugsweise 100 bis 180 °C, und einem Druck von etwa 0,5 bis 6 bar, wobei das Alkylenoxid portionsweise oder kontinuierlich zudosiert wird. Die Menge an Alkylenoxid liegt im allgemeinen bei 1 bis 30 mol, vorzugsweise 2 bis 20 mol und insbesondere 2 bis 15 mol pro mol zu alkoxylierender Verbindung. Das erhaltene Alkoxylat kann im allgemeinen ohne vorherige Abtrennung des Katalysators eingesetzt werden.

Gemäß einer Variante kann man auch so vorgehen, daß man den Katalysator in situ erzeugt. Hierbei geht man so vor, daß man das in Stufe b) erzeugte Erdalkali-Salz zu der Verbindung gibt, die oxalkyliert werden soll, und die Stufen c) und d) dann in Gegenwart dieser Verbindung durchführt.

Die Menge an erfindungsgemäßem Katalysator kann in weiten Grenzen variieren und liegt im allgemeinen bei 0,1 bis 5 Gew.-%, vorzugsweise bei 0,5 bis 3 Gew.-%, bezogen auf das Gewicht der zu oxalkylierten Verbindung.

Als Verbindung, die mit Hilfe des erfindungsgemäßen Katalysators oxalkyliert werden können, kommen alle Verbindungen mit einem aktiven H-Atom in Frage. Auch Verbindungen ohne aktives H-Atom, wie z. B. Fettsäurealkylester lassen sich mit Hilfe des erfindungsgemäßen Katalysators oxalkylieren (Insertion von Ethylenoxid in die Estergruppe).

Aktive H-Atome enthaltende Verbindungen sind zum Beispiel Hydroxylgruppen enthaltende Verbindungen, Aminverbindungen und Säureverbindungen wie Fettsäuren, wobei die erstgenannten bevorzugt sind. Hydroxylgruppen enthaltende Verbindungen sind zum Beispiel Alkohole, Aminoalkohole, Perfluoralkylalkohole, Glykole, Glykolmonoether, Glycerin, Phenole, Kresole und dergleichen, wobei Alkohole bevorzugt sind. Sie können aus nativer Quelle oder aus Synthese-Prozessen stammen, primär, linear oder verzweigt, gesättigt oder ungesättigt, einoder mehrwertig sein, zum Beispiel Oxo-Alkohole, Guerbet-Alkohole, Ziegler-Alkohole, Fettalkohole, Fluoralkohole und dergleichen. Bevorzugte Alkohole sind die primären, geradkettigen oder verzweigten C₃ bis C₂₄-Alkanole, vorzugsweise C₆ bis C₁₈-Alkanole (Fettalkohole) oder Gemische davon, zum Beispiel Gemische aus C₁₂ und C₁₄-Alkanol (C_{12/14}) sowie Perfluoralkohole. Als Beispiele für die bevorzugten Alkohole seien genannt: Butanol, Amylalkohol, Hexanol, Nonanol, Isononylalkohol, Decanol, Undecanol, Isoundecanol, Laurylalkohol, Isotridecylalkohol, Stearylalkohol, Kokosfettalkohol und Gemische davon, ferner 2-Ethylhexanol, 2-Hexyldecanol, 2-Octyldecanol und ähnliche Guerbet-Alkohole.

Als Alkylenoxide werden vorzugsweise Ethylenoxid, Propylenoxid und/oder Butylenoxid eingesetzt, wobei Ethylenoxid bevorzugt ist.

Der erfindungsgemäß hergestellte Alkoxylierungskatalysator weist eine hohe katalytische Aktivität auf und führt in relativ kurzer Reaktionszeit zu einem praktisch vollständigen Umsatz und zu hoher Ausbeute. Das Alkoxylat weist eine enge Homologenverteilung auf und ist farblos und häufig klar und hat damit ein gutes Aussehen.

Insbesondere bei der Ethoxylierung von Fettsäurealkylestern erhält man durch die enge Homologenverteilung und den hohen Umsetzungsgrad einheitlichere Produkte und weniger Nebenprodukte im Vergleich zu einer herkömmlichen Katalyse mit basischen Na- oder K-Verbindungen.

### Beispiel A 1

### Schritt 1 (Ester-Bildung)

In einem beheizbaren Rührbehälter wurden 582 g (=3,00 Mol) C₁₂/C₁₄-Alkohol eingewogen. Nach Aufheizen auf ca. 60 °C wurden unter Rühren in einem Zeitraum von ca. 15 Min. 100 g (=1,00 mol) Bernsteinsäureanhydrid-Schuppen (BSA) zugegeben.
Anschließend wurde in 1/2 Std. aufgeheizt auf 100 °C. Dabei trat eine leicht exotherme Reaktion ein unter Erwärmung des Reaktionsgemisches auf ca. 110 °C.

Nun wurde 1 Std. bei 90 °C nachgerührt und anschließend die Säurezahl kontrolliert (Probe gelöst in IPA/Wasser, potentiometrische Titration mit NaOH). Es wurde eine Säurezahl von 80 [mg KOH/g] gefunden.

### Schritt 2 (Ca-Salz-Bildung)

Nun wurden bei 80 °C in einem Zeitraum von 20 - 30 min 37,0 g Calciumhydroxid-Pulver (=0,50 mol Ca(OH)₂) gemeinsam mit der gleichen Gewichtsmenge Wasser zugesetzt. Dabei war eine leicht exotherme Reaktion zu erkennen.

Anschließend wurde 2 Std. bei 90 - 95 °C nachgerührt zur Sicherstellung der vollständigen Ca-Salz-Bildung. Es entstand eine weiße, bei 25 °C mittelviskose Suspension.
Die Alkalizahl-Bestimmung erfolgt durch potentiometrische Titration mit HClO₄ in Eisessig.
Es wurde eine Alkalizahl 75 [mg KOH/g] gefunden.
Der hier vorliegende Slurry ist eine Vorstufe des erfindungsgemäßen Katalysatorsystems, dessen Aktivität für eine technische Anwendbarkeit noch nicht ausreichend ist.

### Schritt 3 (Teilneutralisation)

Zum vorliegenden basischen C₁₂/C₁₄-Alkohol-Bernsteinsäure-Halbester-Ca-Salz wurden unter Rühren 49 g 40 %ige Schwefelsäure bei 60 °C zugesetzt. Diese Menge entspricht einer 40 %igen Neutralisation der vorliegenden Ca-Basizität.

### Schritt 4 (Trocknung)

Es wurde auf 75 bis max. 90 °C aufgeheizt und unter Vakuum die vorhandenen Mengen Wasser entfernt.

### Beispiel A 2

### Schritt 1 (Ester-Bildung)

In einem beheizbaren Rührbehälter wurden 291 g (=1,50 Mol) C₁₂/C₁₄-Alkohol vorgelegt. Nach Aufheizen auf ca. 60 °C wurden unter Rühren in einem Zeitraum von ca. 15 Min. 100 g (=1,00 mol) Bernsteinsäureanhydrid-Schuppen (BSA) zugegeben.
Anschließend wurde in 1/2 Std. aufgeheizt auf 100 °C. Dabei trat eine exotherme Reaktion ein unter Erwärmung des Reaktionsgemisches auf ca. 115 °C.
Nun wurde 3 Std. bei 90 °C nachgerührt und anschließend die Säurezahl kontrolliert. Es wurde eine Säurezahl von 140 [mg KOH/g] gefunden.

### Schritt 2 (Ca-Salz-Bildung)

Zum vorliegenden sauren Halbester wurden bei 90 °C in einem Zeitraum von 20 - 30 min 37,0 g Calciumhydroxid-Pulver (=0,50 mol Ca(OH)₂) und 74 g Wasser zugesetzt. Dabei war eine leicht exotherme Reaktion wahrnehmbar.
Nun wurde 2 Std. bei 85 - 90 °C nachgerührt. Es entstanden 410 g einer weißen, viskosen Suspension, die bei 35 °C noch fließfähig ist.
Die Alkalizahl-Bestimmung erfolgt durch potentiometrische Titration mit HClO₄ in Eisessig.
Es wurde eine Alkalizahl 130 [mg KOH/g] gefunden.

### Schritt 3 (Teilneutralisation)

Die Teilneutralisation wurde in diesem Beispiel im eigentlichen Ethoxylierungsreaktor durchgeführt. Dazu wurde als zu ethoxylierender Rohstoff 194 g C₁₂/C₁₄-Alkohol (= 1,00 mol) in den Ethoxylierungsreaktor eingefüllt und 2,05 g der genannten Vorstufe (von Schritt 2) zugesetzt. Diese Menge entsprach 0,25 Mol-% Ca, bezogen auf den zu ethoxylierenden Fettalkohol. Dieses Gemisch wurde im Reaktor mit 0,24 g H₂SO₄ (40 %ig) Schwefelsäure teilneutralisiert.

### Schritt 4

Es folgte eine Trocknung bei 90 °C unter Vakuum zur Wasserentfernung bis auf einen Rest-Wassergehalt von ≤ 0,10 %.

### Beispiel A 3

### Schritt 1 (Ester-Bildung)

Es wurde wie in Beispiel A 1 vorgegangen, aber anstelle des C₁₂/C₁₄-Alkohol wurden 744 g (= 2,00 Mol) C₁₂/C₁₄-Alkoholethoxylat mit 4 EO/Mol vorgelegt.
Nach beendetem Schritt 1 wurde eine Säurezahl von 66 [mg KOH/g] gefunden.

### Schritt 2 (Ca-Salz-Bildung)

Für die Ca-Salz-Bildung wurde wie in Beispiel A 1 verfahren. Es entstanden 410g einer weißen, viskosen Suspension, die bei 35 °C noch fließfähig ist.
Die Alkalizahl-Bestimmung liefert einen Wert von 63 [mg KOH/g]

### Schritt 3 (Teilneutralisation)

Die Teilneutralisation erfolgt analog Beispiel A 1 mit 49 g 40 %ige Schwefelsäure bei 60 °C. Diese Menge entspricht einer 40 %igen Neutralisation der vorliegenden Ca-Basizität.

### Schritt 4 (Trocknung)

Trocknung analog Beispiel A 1. Das erhaltene Substrat ist bei 20 °C flüssig und stellt einen guten Katalysator für die Ethoxylierung von Fettalkoholen dar.

### Beispiel A 4

### Schritt 1 (Ester-Bildung)

Es wurde wie in Beispiel A 1 vorgegangen, aber anstelle des C₁₂/C₁₄-Alkohol wurden 1.176g (=6,00 Mol) C₁₂/C₁₄-Alkohol vorgelegt.
Nach beendetem Schritt 1 wurde eine Säurezahl von 44 [mg KOH/g] gefunden.

### Schritt 2 (Ca-Salz-Bildung)

Für die Ca-Salz-Bildung wurde wie in Beispiel A 1 verfahren. Es entstand eine weiße, bei 25 °C mittelviskose Suspension.
Die Alkalizahl-Bestimmung lieferte einen Wert von 43 [mg KOH/g]

### Schritt 3 (Teilneutralisation)

Zum vorliegenden basischen C₁₂/C₁₄-Alkohol-Bernsteinsäure-Halbester-Ca-Salz wurden unter Rühren 49,0 g 50 %ige Schwefelsäure bei 60 °C zugesetzt. Diese Menge entspricht einer 50 %igen Neutralisation der vorliegenden Ca-Basizität.

### Schritt 4 (Trocknung)

Trocknung analog Beispiel A 1. Das erhaltene Substrat ist bei 20 °C flüssig und ist ein einsatzfähiger Katalysator zur Herstellung von Fettalkohol-Ethoxylaten.

### Beispiel A 5

### Herstellung eines Ca-haltigen Katalysators auf Fluoralkohol-Basis

### Schritt 1 (Ester-Bildung)

In einem beheizbaren Rührbehälter wurden 1.350 g (=3,00 Mol) eines Perfluoralkylethanol-Homologengemisches der allgemeinen Summenformel F-(CF₂)ₙ-(CH₂)ₘ-OH vorgelegt (Mischung aus n = 6 bis 16, m = 2).
Nach Aufheizen auf ca. 60 °C wurden unter Rühren in einem Zeitraum von ca. 15 Min. 100 g (=1,00 mol) Bernsteinsäureanhydrid-Schuppen (BSA) zugegeben. Anschließend wurde in 1/2 Std. weiter aufgeheizt auf 90 °C. Dabei trat eine leicht exotherme Reaktion ein unter Erwärmung des Reaktionsgemisches auf ca. 110 - 115 °C.
Nun wurde 2 Std. bei 100 °C nachgerührt und anschließend die Säurezahl kontrolliert (Probe gelöst in IPA/Wasser, potentiometrische Titration mit NaOH). Es wurde eine Säurezahl von 39 [mg KOH/g] gefunden.

### Schritt 2 (Ca-Salz-Bildung)

Nun wurden 80 °C in einem Zeitraum von 20 - 30 min 37,0 g Calciumhydroxid-Pulver (= 0,50 mol Ca(OH)₂) gemeinsam mit der gleichen Gewichtsmenge Wasser zugesetzt. Dabei ist eine leicht exotherme Reaktion zu erkennen.
Anschließend wurde 2 Std. bei 90 - 95 °C zur Ca-Salz-Bildung nachgerührt. Es entstand eine weiße, viskose Suspension, die bei 25 °C noch fließfähig ist.
Die Alkalizahl-Bestimmung erfolgt durch potentiometrische Titration mit HClO₄ in Eisessig.
Es wurde eine Alkalizahl 37 [mg KOH/g] gefunden.

### Schritt 3 (Teilneutralisation)

Zur Teilneutralisation wurde zum vorliegenden basischen PerfluoralkylethanolBernsteinsäure-Halbester-Ca-Salz 49 g 40 %ige Schwefelsäure zugesetzt. Diese Menge entspricht einer 40 %igen Neutralisation der vorliegenden Ca-Basizität. Es folgte eine Trocknung bei 75 °C unter Vakuum zur Entfernung der restlichen Mengen Wasser.

### Zusammenfassung der Katalysator-Herstellbeispiele:

| Beispiel | | Molverhältnis von Alkohol : BSA : Ca | Teilneutralisation mit H₂SO₄ x % der Ca-Basizität |
|---|---|---|---|
| A 1 | C₁₂/C₁₄-Alkohol-Bernsteinsäure-Halbester-Ca-Salz | 3 : 1 : 0,5 | 40 % |
| A 2 | C₁₂/C₁₄-Alkohol-Bernsteinsäure-Halbester-Ca-Salz | 1,5 : 1 : 0,5 | 40 % im Ethoxylierungsreaktor |
| A 3 | C₁₂/C₁₄-Alkohol + 4 EO-Bernsteinsäure-Halbester-Ca-Salz | 2 : 1 : 0,5 | 40 % |
| A 4 | C₁₂/C₁₆-Alkohol-Bernsteinsäure-Halbester-Ca-Salz | 6 : 1 : 0,5 | 50 % |
| A 5 | Perfluoralkylethanol-Bernsteinsäure-Halbester-Ca-Salz | 3 : 1 : 0,5 | 40 % |

### Beispiele für die Verwendung der erfindungsgemäßen Katalysatoren für die Ethoxylierung

### Beispiel E 1

### Variante a

In einen Druckreaktor aus Glas wurden 194 g C_{12/14}-Alkohol (= 1,00 Mol) und 3,6g Katalysator-Typ A 1 (= 0,25 Mol-% Ca) eingewogen und nach mehrmaliger Spülung des Reaktionsraumes mit Stickstoff bis 160°C aufgeheizt. Bei dieser Temperatur wurde mit der Dosierung von Ethylenoxid begonnen. Nach einer Startphase von ca. 15 Min. begann die Reaktion des Ethylenoxids, so daß insgesamt 176 g Ethylenoxid (= 4 Mol EO/Mol) bei 160 - 170 °C in etwa 2 Std. umgesetzt wurden.

### Beispiel E 2

### Variante b

Bei dieser Variante erfolgt die Teilneutralisationsstufe im Ethoxylierungsreaktor, wie im Beispiel A 2 schon beschrieben:
Dazu wurde als zu ethoxylierender Rohstoff 194 g C₁₂/C₁₄-Alkohol (= 1,00 mol) in den Ethoxylierungsreaktor eingefüllt und 2,05 g der genannten Vorstufe (von Beispiel A 2, Schritt 2) zugesetzt. Diese Menge entspricht 0,25 Mol-% Ca, bezogen auf den zu ethoxylierenden Fettalkohol.

Das resultierende Gemisch wurde im Ethoxylierungsreaktor mit 0,24 g H₂SO₄ (40 % ig) Schwefelsäure teilneutralisiert. Es folgte eine Trocknung bei 90 °C unter Vakuum zur Wasserentfernung bis auf einen Rest-Wassergehalt von ≤ 0,10 %.

Anschließend wurde bis 160 °C aufgeheizt und mit der Dosierung von Ethylenoxid begonnen. Nach einer Startphase von ca. 15 Min. wurden insgesamt 176g Ethylenoxid (= 4 Mol EO/Mol) in etwa 2 Std. bei 160 - 170 °C zudosiert.

Diese Beispiele E 1 und E 2 sowie die analog durchgeführten erfindungsgemäßen Beispiele E 3 bis E 9 sind in der folgenden Tabelle zusammengefaßt. Diese Tabelle enthält außerdem die Vergleichsbeispiele V 1 bis V 4.

### Tabellarische Zusammenfassung der Ethoxylierungs-Beispiele

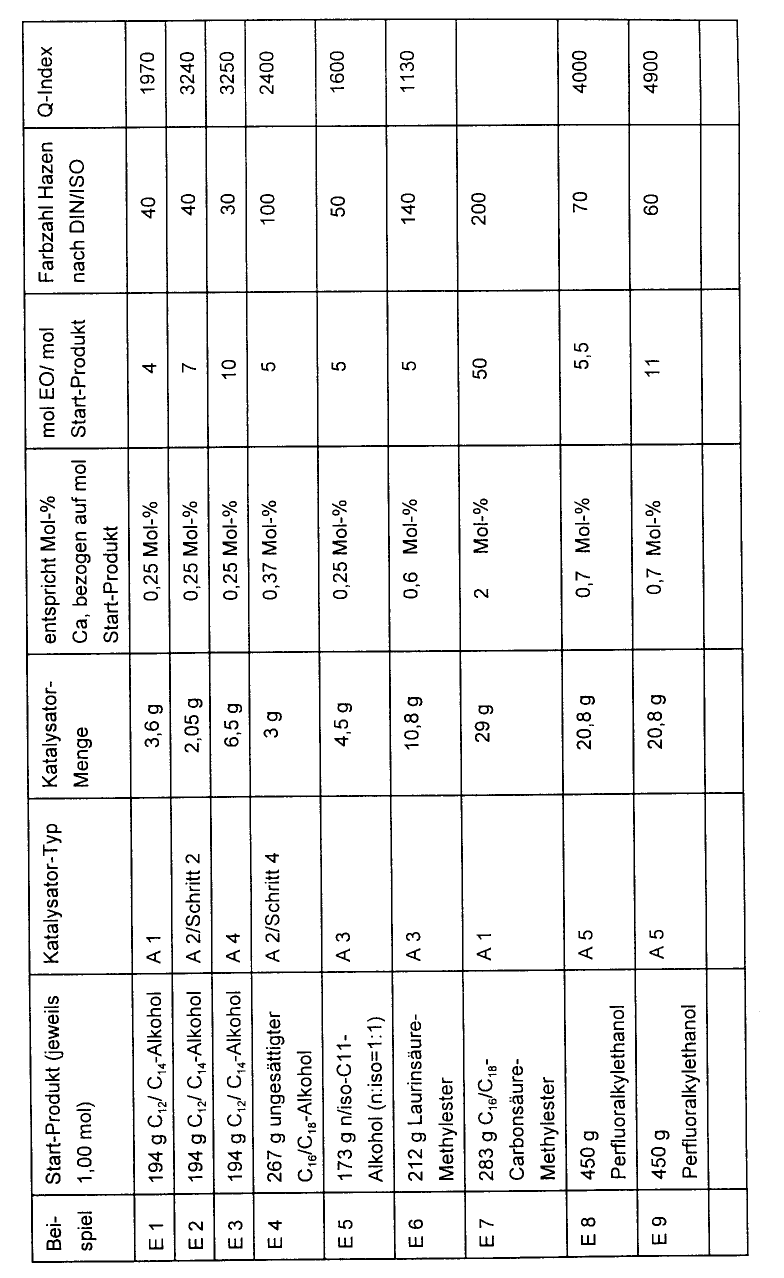

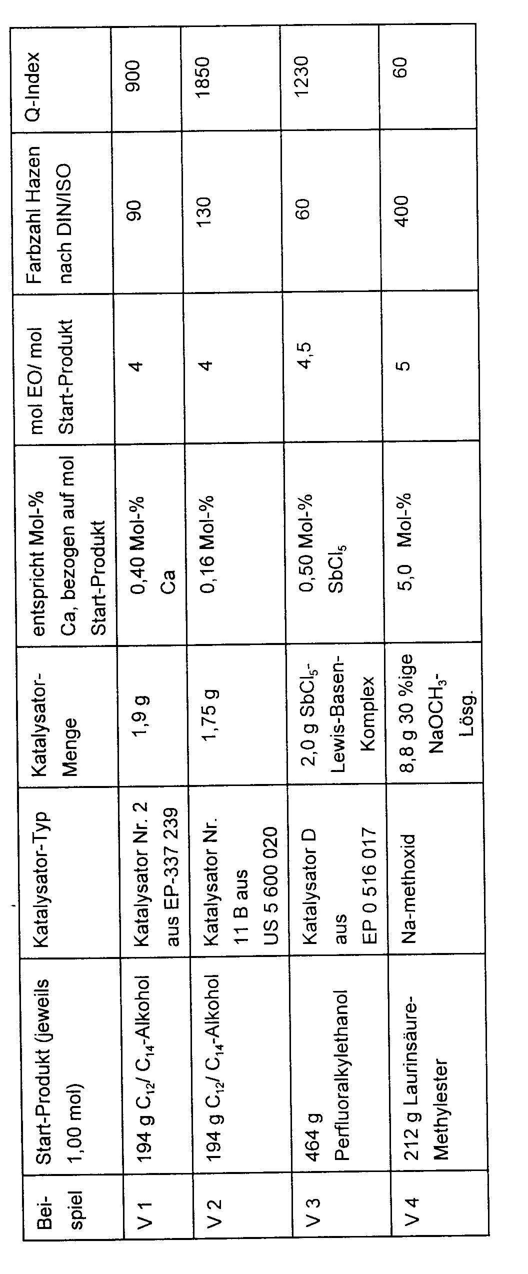

Der Q-Index gibt das Maß der Homologenverteilung an. Es gilt die Beziehung Q = n · p², wobei n die durchschnittliche Adduktzahl (mittlerer Ethoxylierungsgrad) und p den Prozentsatz des Adduktes mit bestimmten EO-Grad, das überwiegend gebildet wird, bedeuten. Ein großer Q-Wert bedeutet somit eine enge Bandbreite der Homologenverteilung.

## Patentansprüche

1. Alkoxylierungskatalysator, hergestellt durch
a) Umsetzung von einem Mol einer Dicarbonsäure der Formel
HOOC-(CH₂)ₐ-COOH,
wobei a eine Zahl von 1 bis 4 bedeutet, mit 1,5 bis 8 mol eines Alkohols der Formel
R-(OA)ₓ-OH
worin R C₄-C₂₂-Alkyl, C₄-C₂₂-Alkenyl oder eine Gruppe der Formel F-(CF₂)ₙ-(CH₂)ₘ-, n eine ganze Zahl von 6 bis 16, m eine ganze Zahl von 1 bis 4, x eine Zahl von 0 bis 6 und A -C₂H₄- oder -C₃H₆- bedeutet, unter Bildung des entsprechenden Dicarbonsäurehalbesters,
b) Bildung eines Erdalkali-Salzes des Dicarbonsäurehalbesters durch Zugabe von Wasser und 0,45 bis 0,55 mol einer basischen Erdalkaliverbindung pro Mol ursprünglich eingesetzter Dicarbonsäure,
c) Teilneutralisation des Erdalkali-Salzes durch Zugabe von 0,25 bis 0,7 mol H₂SO₄ pro Mol ursprünglich eingesetzter basischer Erdalkaliverbindung und
d) Entfernung des vorhandenen Wassers bei einer Temperatur unter 100 °C.

2. Verfahren zur Oxalkylierung von Verbindungen mit einem aktiven H-Atom sowie Fettsäurealkylestern, dadurch gekennzeichnet, daß man in Gegenwart eines Oxalkylierungskatalysators gemäß Anspruch 1 arbeitet.
